# EUROPEAN PATENT SPECIFICATION

(11) **EP 1 765 314 B1**
(45) Date of publication and mention of the grant of the patent: **01.05.2019**
(21) Application number: 05749393.4
(22) Date of filing: 07.06.2005
(51) Int. Cl.: A61P 19/02, A61K 47/10, A61K 9/00, A61K 31/05, A61K 36/185, A61K 31/047, A61K 31/352

(54) **PHARMACEUTICAL COMPOSITIONS FOR THE TREATMENT OF DISEASE AND SYMPTOMS IN RHEUMATOID ARTHRITIS**
PHARMAZEUTISCHE ZUSAMMENSETZUNGEN ZUR BEHANDLUNG VON ERKRANKUNGEN UND SYMPTOMEN VON RHEUMATOIDER ARTHRITIS
COMPOSITIONS PHARMACEUTIQUES DESTINEES AU TRAITEMENT DE LA POLYARTHRITE RHUMATOÏDE ET DE SES SYMPTOMES

(30) Priority: 08.06.2004 GB 0412753
(43) Date of publication of application: 28.03.2007
(73) Proprietor: GW Pharma Limited, Histon Cambridge CB24 9BZ (GB)
(72) Inventor: ROBSON, P., Pharma Limited, Salisbury,Wiltshire SP4 0JQ (GB); GUY, G., Pharma Limited, Salisbury,Wiltshire SP4 0JQ (GB)
(74) Representative: HGF Limited
(86) International application number: PCT/GB2005/002233
(87) International publication number: WO 2005/120478

(56) References cited:
- WO-A-02/064109
- WO-A-02/069993
- "Cannabis-based medicines - GW Pharmaceuticals. High CBD, high THC, medicinal cannabis - GW Pharmaceuticals, THC:CBD" DRUGS IN R AND D 2003 NEW ZEALAND, vol. 4, no. 5, 2003, pages 306-309, XP009048624 ISSN: 1174-5886
- A. M. Malfait ET AL: "The nonpsychoactive cannabis constituent cannabidiol is an oral anti-arthritic therapeutic in murine collagen-induced arthritis", Proceedings of the National Academy of Sciences, vol. 97, no. 17, 15 August 2000 (2000-08-15), pages 9561-9566, XP055202324, ISSN: 0027-8424, DOI: 10.1073/pnas.160105897

## Description

The present invention relates to the use of a combination of cannabinoids cannabidiol (CBD) and delta-9-tetrahydrocannabinol (THC) for use as a disease modifying treatment of rheumatoid arthritis. The cannabinoids are in a ratio of substantially 1:1 and the dose taken by the patient is in the range of 5-25 mg of each cannabinoid per day. The cannabinoids are administered sublingually or buccally.

### BACKGROUND TO THE INVENTION

Arthritis is a painful condition of the joints. There are different types of the disease yet all cause pain and inflammation of the joints and are often degenerative in nature. Some of the most common types of arthritis are osteoarthritis and rheumatoid arthritis.

Osteoarthritis is a disease that affects the joints of around 8 in 10 people over the age of 50. Osteoarthritis is caused by the joint cartilage becoming thin and uneven over time and can in some cases wear out completely. In addition to the wearing out of the joints, the joint capsule can become thicker and in consequence there is an increase in the amount of synovial fluid that is generated. This in turn causes the joint to swell. Bony spurs may also grow in the affected area causing inflammation in the affected tissues. Osteoarthritis can involve all joints in the body, but is most commonly found in the fingers, knees, hips and spine.

Rheumatoid arthritis is a systemic disease, which can affect the entire body and is one of the most common forms of arthritis. It is characterised by inflammation of the membranes that line a joint, which in turn causes pain, stiffness, warmth, redness and swelling to the area. The small joints of the fingers and hands are most seriously affected but the condition can spread to involve the wrists, elbows, shoulders and other joints. The inflamed joint lining can also invade and damage bone and cartilage when inflammatory cells release enzymes that are able to digest bone and cartilage. The inflamed joint can lose its shape and alignment, resulting in pain and loss of movement. It is typically chronic and can flare-up at intervals.

In addition to the pain and inflammation experienced in the affected joints, rheumatoid arthritis can cause loss of appetite and weight, lethargy, muscle and tendon pain, fever, lumps under the skin (rheumatoid nodules) and severe eye inflammation. There are many complications including anaemia, pericarditis, vasculitis and Raynaud's phenomenon.

The cause of rheumatoid arthritis is not yet known. However, it is known that rheumatoid arthritis is an autoimmune disease. The body's natural immune system does not operate as it should, resulting in the immune system attacking healthy joint tissue and causing inflammation and subsequent joint damage. The disease could be triggered by an infection in some people who have an inherited tendency for the disease that prompts the immune system to form damaging aggregates of antigen and antibody immune complexes.

Early in the disease, people may notice general fatigue, soreness, stiffness and aching. Pain and swelling may occur in the same joints on both sides of the body and will usually start in the hands or feet. Rheumatoid arthritis affects the wrist and many of the hand joints, but usually not the joints that are closest to the fingernails (except the thumb). Rheumatoid arthritis can also affect elbows, shoulders, neck, knees, hips and ankles. It tends to persist over prolonged periods of time, and over time, the inflamed joints may become damaged.

Treatment of rheumatoid arthritis is limited to the control of inflammation and the relief of pain by means of rest, splinting of the inflamed joint, physiotherapy, and the use of anti-inflammatory and pain killing drugs. The treatment methods focus on relieving pain, reducing inflammation, stopping or slowing joint damage, and improving the patient's well being.

The current medications that are provided to patients with rheumatoid arthritis can be divided into two groups;
1. Symptomatic medications, such as non-steroidal anti-inflammatory drugs (NSAIDs) and aspirin, analgesics, and corticosteroids. These drugs help reduce joint pain, stiffness and swelling. Symptomatic medications may be used in combination with disease-modifying anti-rheumatic drugs.
2. Disease-modifying anti-rheumatic drugs (DMARDs), include low doses of methotrexate, leflunomide, D-Penicillamine, sulfasalazine, gold therapy, minocycline, azathioprine, hydroxychloroquine (and other antimalarials), cyclosporine and biologic agents.

In addition to drug therapy, treatment most often involves some combination of exercise, rest, joint protection, and physical and occupational therapy. Surgery can be an option for joints that are severely damaged and painful. A balance of rest and exercise can help conserve energy and maintain a range of motion and use of the joints.

The use of cannabis as a medicine has long been known and during the 19^{th} Century preparations of cannabis were recommended as a hypnotic sedative which were useful for the treatment of hysteria, delirium, epilepsy, nervous insomnia, migraine, pain and dysmenorrhoea.

Until recent times the administration of cannabis to a patient could only be achieved by preparation of cannabis by decoction in ethanol, which could then be swallowed or by the patient inhaling the vapours of cannabis by smoking the dried plant material. Recent methods have sought to find new ways to deliver cannabinoids to a patient including those which bypass the stomach and the associated first pass effect of the liver which can remove up to 90% of the active ingested dose and avoid the patient having to inhale unhealthy tars and associated carcinogens into their lungs.

Such dosage forms include administering the cannabinoids to the sublingual or buccal mucosae, inhalation of a cannabinoid vapour by vaporisation or nebulisation, enemas or solid dosage forms such as gels, capsules, tablets, pastilles and lozenges.

In 1988 a study was undertaken in order to determine the analgesic and anti-inflammatory activity of various cannabinoids and cannabinoid pre-cursors. Oral administration of CBD was found to be the most effective at inhibition of PBQ-induced writhing in mice. THC and CBN were found to be least effective at reducing analgesia and inflammation (Formukong et al., 1988).

Holdcroft *et al*. have shown that cannabinoids can have analgesic and possible anti-inflammatory properties. Administration of 50mg of THC to a patient with Mediterranean fever resulted in a highly significant reduction in the amount of analgesia that the patient required (Holdcroft *et al*., 1997a).

A follow-on publication by the same authors examined the oral administration of oil of cannabis. The capsules containing 5.75% THC, 4.73% CBD and 2.42% CBN were administered to a patient with familial Mediterranean fever. During the 3 weeks of active treatment there was a decrease in the amount of escape medication (morphine) required by the patient (Holdcroft *et al*., 1997b). There were no changes in the measured inflammatory markers.

It has previously been shown by Feldmann *et al*. (International patent application WO 99/52524) that pure CBD can be used to treat inflammatory diseases such as rheumatoid arthritis or Crohn's disease. Inflammatory diseases involve a complex interaction between several components such as Interleukins, TNF-α and nitric oxide. The data presented by Feldmann *et al*. describes the inhibition of TNF-α and nitric oxide production by CBD. This cannabinoid was also shown to suppress arthritis in a dose dependant manner in a collagen induced arthritis model in mice. In addition, Malfait *et al* (2000) described the use of CBD in a mouse model of arthritis.

There is considerable literature concerning the immune modulating effects of constituents of cannabis a review of these was undertaken by Klein (Klein, 1998).

The use of different ratios of cannabinoids such as THC or CBD or their propyl variants, tetrahydrocannabinovarin (THCV) and cannabidivarin (CBDV), in the treatment of different diseases and conditions has previously been described by the applicant in their UK patent application GB2377633.

Specific ratios of THC and CBD or THCV and CBDV were reported to have been useful in the treatment or management of specific diseases or medical conditions. The following table details some of these areas.

| **Product Group Area** | **Ratio THC:CBD** | **Target Therapeutic** |
|---|---|---|
| High THC | >95:5 | Cancer pain; Migraine; Appetite stimulation. |
| Even ratio | 50:50 | Multiple sclerosis; Spinal cord injury; Peripheral neuropathy; Neurogenic pain. |
| Broad ratio CBD | <25:75 | Rheumatoid arthritis; inflammatory bowel disease. |
| High CBD | <5:95 | Psychotic disorders (schizophrenia); Epilepsy; Movement disorders; Stroke; Head injury; Disease modification in rheumatoid arthritis and other inflammatory conditions; Appetite suppression. |

Formulations containing specific, defined ratios of cannabinoids may be formulated from pure, synthetic cannabinoids or from extracts derived from the cannabis plant in combination with pharmaceutical carriers and excipients.

A major disadvantage with the currently available drug therapies to treat arthritis is that the patient often has to take a combination of drugs in order to treat the symptoms of the disease such as the pain and associated inflammation, and at the same time the patient has to take a drug in order to modify the disease.

At present there are no known medications to treat the symptoms of pain and inflammation and at the same time act as disease modifying anti-rheumatic drugs.

Surprisingly it has been found that the use of a cannabis based medicine extract that contains approximately equal amounts of the cannabinoids delta-9-tetrahydrocannabinol (THC) and cannabidiol (CBD) can be used to both modify rheumatoid arthritis disease and treat the symptoms of pain and inflammation caused by the disease.

An important benefit of the use of the medication described in the present invention is that both the disease and the symptoms of the disease can be treated by the same medication. This in turn has numerous benefits which include a greater degree of flexibility for the patient as they will have to adhere to a less strict drug regime, the patient is also likely to experience less side effects as there will be less potentially harmful interaction between combined drug therapies.

### SUMMARY OF INVENTION

According to the first aspect of the present invention there is provided a combination of cannabinoids cannabidiol (CBD) and delta-9-tetrahydrocannabinol (THC) for use as a disease modifying treatment of rheumatoid arthritis wherein the ratio of CBD:THC is substantially 1:1, and wherein the dose taken by a patient is in the range of 5-25mg of each cannabinoid per day sublingually or buccally.

Preferably the use of the combination of cannabinoids further comprises the treatment of one or more of the symptoms of rheumatoid arthritis. More preferably these symptoms include pain, inflammation or improvement in quality of sleep.

Preferably the ratio of CBD:THC is 0.93:1.

Preferably the combination of cannabinoids are packaged for delivery sublingually or buccally, preferably as a sublingual or buccal spray. Advantageously the pharmaceutical formulation further comprises one or more carrier solvent/s. Preferably the carrier solvents are ethanol and/or propylene glycol. More preferably the ratio of ethanol to propylene glycol is between 4:1 and 1:4. More preferably still the ratio is 1:1.

Favourably the dose is formulated such that a patient is able to titrate their dose. Dose ranges are preferably in the range of between 5 and 25mg of each cannabinoid, more preferably in the range of 10 to 20mg of each cannabinoid, preferably in the range of 12 to 14mg of each cannabinoid more preferably still in the range of 12.5 to 13.5 mg of each cannabinoid.

The administration of a combination of cannabinoids such as THC and CBD could be administered to a patient either at the same time, wherein the cannabinoids would be contained in the same formulation. The cannabinoids could also be administered at separate times for example; a formulation containing CBD could be administered to a patient at a fixed time prior to a formulation containing THC in order to ameliorate some of the side effects of THC, which CBD is known to improve or vice versa. The two cannabinoids could also be administered consecutively to a patient if required.

Preferably the invention provides a combination of cannabinoids, which are present as one or more cannabis based medicine extract/s (CBME/s). In one embodiment the CBME/s are produced by extraction with supercritical or subcritical CO₂. In an additional embodiment the CBME/s are produced by extraction from plant material by volatilisation with a heated gas. Preferably the CBME/s contain all of the naturally occurring cannabinoids in the plant material. Alternatively synthetic or highly purified isolates of the cannabinoids can be used.

### SPECIFIC DESCRIPTION

A cannabis based medicine extract (CBME) was prepared as outlined in Example 1 and contained approximately equal amounts of the cannabinoids THC and CBD and this was administered to patients with chronic rheumatoid arthritis with pain as a secondary condition. The administration of this combination of cannabinoids could possibly reduce the pain and inflammation caused by the rheumatoid arthritis but unexpectedly the cannabis based medicine extract containing approximately equal quantities of THC and CBD also produced a disease modifying effect in the patients with rheumatoid arthritis.

The features of the invention are illustrated further by reference to the following examples together with the accompanying Figures in which:
Figure 1 shows an HPLC chromatographic profile of a CBD-containing cannabis based medicine extract (CBME).
Figure 2 shows an HPLC chromatographic profile of a THC-containing cannabis based medicine extract (CBME).
Figure 3 shows an HPLC chromatographic profile of a cannabis based medicine extract (CBNE) containing substantially equal quantities of CBD and THC.

### Example 1:

### Preparation of cannabis based medicine extracts (CBME)

Medicinal cannabis was produced and prepared with reference to the method disclosed in WO 02/064109 (Example 15). The resulting plant material was processed as described in the flow chart below. The process of manufacture of a High THC or High CBD cannabis based medicine extract is described.
Medicinal Cannabis (High THC or High CBD) ↓ Chopping to predominantly 2-3mm ↓ Heating at 100-150°C for sufficient time to decarboxylate the acid form of cannabinoids to produce neutral cannabinoids ↓ Extraction with a specific volume of liquid carbon dioxide over 6 to 8 hours ↓ Removal of CO₂ by depressurisation to recover crude extract ↓ Winterisation followed by chilling (-20°c/48h) to precipitate unwanted waxes ↓ Removal of unwanted waxy material by cold filtration ↓ Removal of ethanol from the filtrate by thin film evaporation under reduced pressure

The resulting extract is referred to as a cannabis based medicinal drug extract and is also classified as a Botanical Drug Substance according to the US Food and Drug Administration Guidance for Industry Botanical Drug Products.

The quantity of cannabinoid in the CBME can be accurately assessed by way of measurement by HPLC with reference to the method disclosed in WO 02/064109 (Example 16).

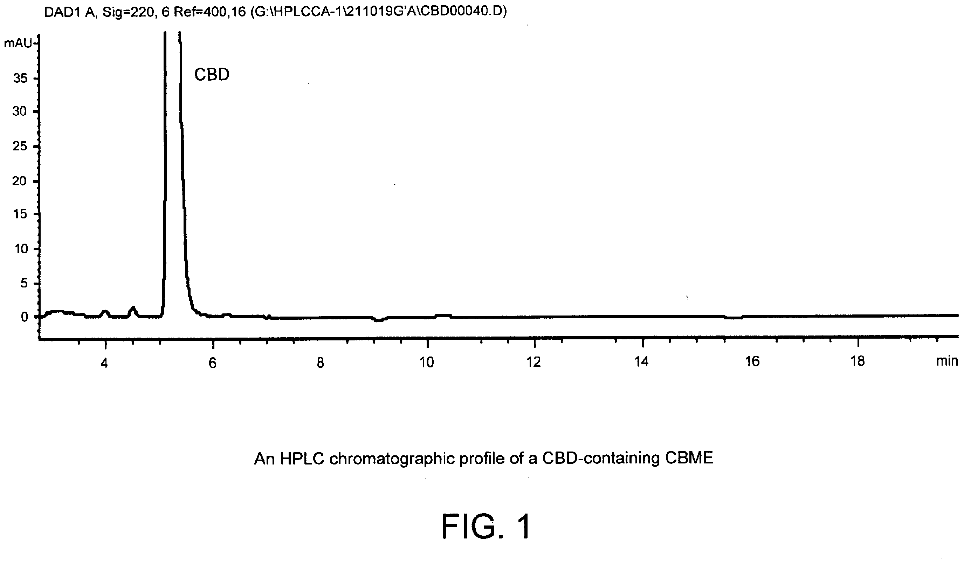

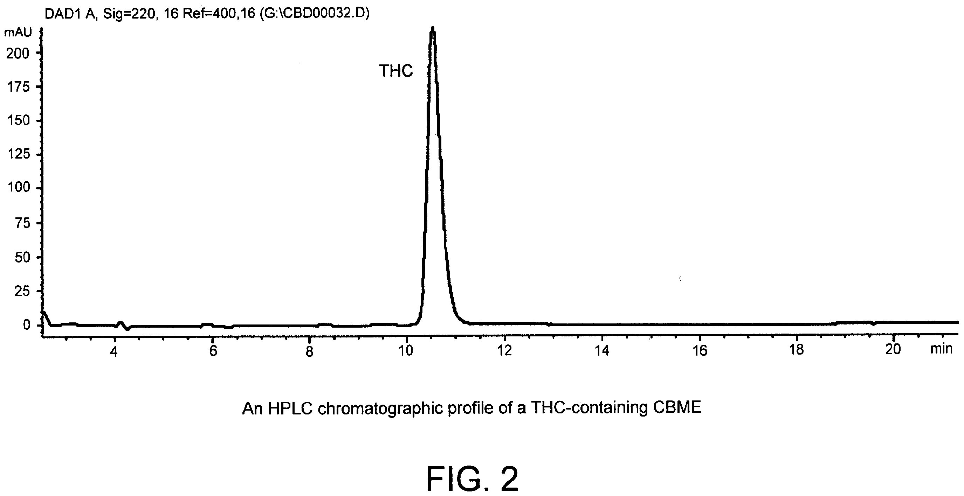

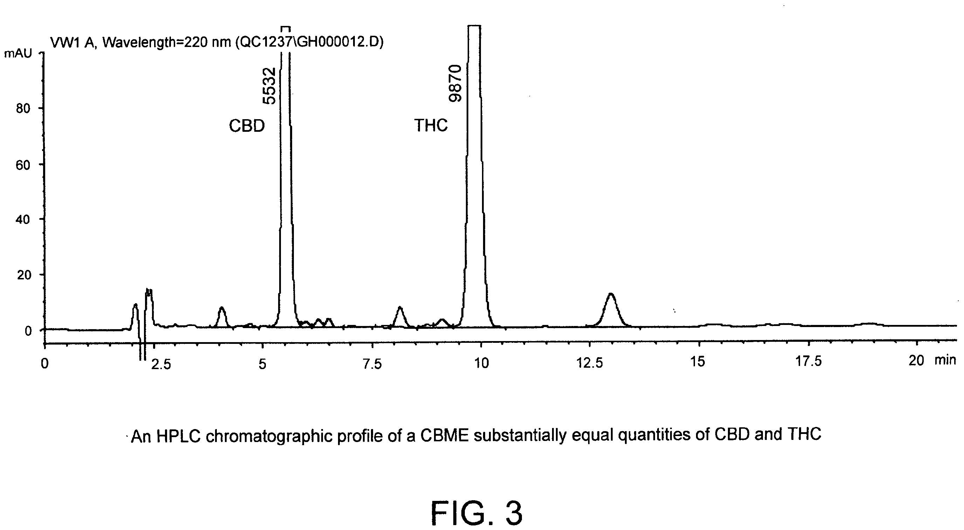

An example of an HPLC chromatogram of a CBD-containing CBME produced using a high CBD medicinal cannabis plant extracted with CO₂ is shown in Figure 1. An example of an HPLC chromatogram of a THC-containing CBME produced using a high THC medicinal cannabis plant extracted with CO₂ is shown in Figure 2. An example of an HPLC chromatogram containing the relevant ratios of THC and CBD CBMEs is shown in Figure 3.

The invention has been exemplified with reference to THC and CBD yet it is clear to a man skilled in the art that the pharmacological similarities between THC and THCV and CBD and CBDV are such that similar results could be produced using the cannabinoids THCV and CBDV in place of or in addition to THC and CBD.

### Example 2:

### Assessment of the efficacy of a cannabis based medicine extract by way of a clinical trial in human rheumatoid arthritis patients.

A seven week, multi-centre, double blind, randomised, parallel group study was undertaken in order to evaluate the efficacy a cannabis based medicine extract on pain in rheumatoid arthritis. The cannabis based medicine extract contained delta-9-tetrahydrocannabinol (THC) at a concentration of 27mg/ml and cannabidiol (CBD) at a concentration of 25mg/ml in ethanol:propylene glycol (50:50) excipient. The cannabis based medicine extract was presented in a pump action spray where each activation delivers 100µl of spray, containing THC (2.7mg) and CBD (2.5mg).

The subjects in the study were randomised equally to either the cannabis based medicine extract or a placebo. The placebo matched the appearance, smell and taste of the active formulation, but containing no active components, in ethanol:propylene glycol (50:50) excipient. Again the placebo was presented in a pump action spray where each activation delivers 100µl of spray.

Patients were screened to determine eligibility at visit 1 and baseline assessments were taken at this time. The patients returned 2 weeks later for visit 2 at which point they were randomised into one of the two groups. The study medication was administered as an evening dose only and patients were asked to titrate their dose until they obtained optimum efficiency.

After 2 weeks titration on the medication the patients returned for visit 3, at this point the patient confirmed the dose that they were to take for the remaining 3 weeks of the study.

The dose of medication that each patient took varied but was in the range of 5-25mg each of THC and CBD, with the majority of patients receiving between 10 and 20mg each of THC and CBD. The average dose that each patient titrated to was 13.5mg THC and 12.5mg CBD.

After 5 weeks on the study medication the patients returned to make visit 4. All baseline assessments were repeated at this stage.

Efficacy assessments were considered as part of the study. Diary card self-assessments were recorded by each patient on a daily basis for morning pain at rest and on movement, morning stiffness and quality of sleep. Short form McGill Questionnaires were completed at visits 1 and 4 in order to compare changes in intensity of pain, intensity of pain at present, pain at present and global impression of change.

A Disease Activity Score was calculated at visits 1 and 4 from a 28 joint count, erythrocyte sedimentation rate and global disease activity score.

Assessments of the use of rescue analgesia, adverse events, blood chemistry and vital signs were all recorded at visits 1 and 4 in order to consider any changes.

### Results:

Some of the data collated from this study is described below.

### Comparison of morning pain at rest in patients with rheumatoid arthritis when administered a cannabis based medicine extract containing THC at a concentration of 27mg/ml and CBD at a concentration of 25mg/ml

The efficacy of a cannabis based medicine extract was assessed as described above and the degree of morning pain at rest was recorded by self assessment on a daily basis. The data was collated and statistical analysis was undertaken. Patients assessed morning pain at rest on a scale of 0 (no pain) to 10 (extremely bad pain). Tables 1 and 2 illustrate the results.

**Table 1:**

| | | **THC:CBD (27mg/ml:25mg/ml) (N=31)** | **Placebo (N=27)** |
|---|---|---|---|
| **Baseline** | Mean | 5.5 | 5.6 |
| | Std Dev | 1.8 | 1.6 |
| | Minimum | 2 | 3 |
| | Median | 5.3 | 5.3 |
| | Maximum | 10 | 9 |
| **Week 1** | Mean | 4.6 | 5.2 |
| | Std Dev | 1.6 | 1.6 |
| | Minimum | 1 | 3 |
| | Median | 4.6 | 4.9 |
| | Maximum | 9 | 9 |
| **Week 1 - change from baseline** | Mean | -0.9 | -0.4 |
| | Std Dev | 1.1 | 1.0 |
| | Minimum | -5 | -3 |
| | Median | -0.6 | -0.4 |
| | Maximum | 1 | 2 |
| **Week 2** | Mean | 3.7 | 4.3 |
| | Std Dev | 1.9 | 1.9 |
| | Minimum | 1 | 1 |
| | Median | 3.7 | 4.2 |
| | Maximum | 9 | 10 |
| **Week 2** - **change from baseline** | Mean | -1.7 | -1.1 |
| | Std Dev | 1.8 | 1.6 |
| | Minimum | -6 | -5 |
| | Median | -1.3 | -0.8 |
| | Maximum | 1 | 2 |
| **Week 3** | Mean | 3.7 | 4.4 |
| | Std Dev | 1.8 | 1.7 |
| | Minimum | 0 | 0 |
| | Median | 3.6 | 4.3 |
| | Maximum | 8 | 8 |
| **Week 3** - **change from baseline** | Mean | -1.8 | -1.1 |
| | Std Dev | 1.8 | 1.7 |
| | Minimum | -7 | -5 |
| | Median | -1.3 | -0.8 |
| | Maximum | 0 | 2 |
| **Week 4** | Mean | 3.5 | 4.4 |
| | Std Dev | 1.8 | 1.9 |
| | | | |
| | Minimum | 0 | 1 |
| | Median | 3.3 | 4.4 |
| | Maximum | 9 | 8 |
| **Week 4** - **change from baseline** | Mean | -2.0 | -1.0 |
| | Std Dev | 1.9 | 1.7 |
| | Minimum | -7 | -5 |
| | Median | -1.6 | -0.8 |
| | Maximum | 1 | 3 |
| **Week 5** | Mean | 3.4 | 4.3 |
| | Std Dev | 1.8 | 1.9 |
| | Minimum | 0 | 0 |
| | Median | 3.1 | 4.3 |
| | Maximum | 8 | 8 |
| **Week 5** - **change from baseline** | Mean | -2.0 | -1.1 |
| | Std Dev | 2.0 | 1.9 |
| | Minimum | -7 | -5 |
| | Median | -1.8 | -1.0 |
| | Maximum | 1 | 2 |
| **Week 6** | Mean | 3.6 | 4.6 |
| | Std Dev | 1.7 | 0.5 |
| | Minimum | 2 | 4 |
| | Median | 3. 0 | 4.9 |
| | Maximum | 6 | 5 |
| **Week 6** - **change from** | Mean | -2.3 | -0.2 |
| | Std Dev | 0.9 | 1.3 |
| **baseline** | Minimum | -3 | -2 |
| | Median | -2.0 | 0.5 |
| | Maximum | -2 | 1 |
| **End Point** | Mean | 3.5 | 4.7 |
| | Std Dev | 1.7 | 2.1 |
| | Minimum | 0 | 0 |
| | Median | 3.1 | 4.1 |
| | | | |
| | Maximum | 8 | 9 |
| **End Point** - **change from baseline** | Mean | -2.0 | -0.9 |
| | Std Dev | 1.9 | 1.7 |
| | Minimum | -7 | -5 |
| | Median | -1.5 | -0.7 |
| | Maximum | 1 | 2 |

Statistical analysis of this data is shown in Table 2.

**Table 2:**

| **THC:CBD (27mg/ml: 25mg/ml)** | | **Placebo** | | | | |
|---|---|---|---|---|---|---|
| LS Mean | s.e. | LS Mean | s.e. | Difference | 95% CI | p-value |
| -2.01 | 0.30 | -0.87 | 0.32 | -1.13 | [-2.02, -0.25] | 0.013 |

The LS Mean figure is the mean change from the baseline adjusted score, a negative difference indicates a benefit.

Tables 1 and 2 demonstrate that the administration of THC:CBD (27mg/ml:25mg/ml) to patients suffering pain in rheumatoid arthritis results in a statistically significant reduction in morning pain at rest when compared to the placebo.

### Comparison of quality of sleep in patients with rheumatoid arthritis when administered a cannabis based medicine extract containing THC at a concentration of 27mg/ml and CBD at a concentration of 25mg/ml

The efficacy of a cannabis based medicine extract was assessed as described above and the quality of sleep experienced by the patient was recorded by self assessment on a daily basis. The data was collated and statistical analysis was undertaken. Patients assessed quality of sleep on a scale of 0 (very good) to 10 (very bad). Tables 3 and 4 illustrate the results.

**Table 3:**

| | | **THC:CBD (27mg/ml:25mg/ml) (N=31)** | **Placebo (N=27)** |
|---|---|---|---|
| **Baseline** | Mean | 5.7 | 5.8 |
| | Std Dev | 1.9 | 1.8 |
| | Minimum | 2 | |
| | Median | 5.5 | 6.0 |
| | Maximum | 10 | 10 |
| **Week 1** | Mean | 4.7 | 5.3 |
| | Std Dev | 1.8 | 1.8 |
| | Minimum | 2 | 2 |
| | Median | 4.9 | 5.4 |
| | Maximum | 8 | 10 |
| **Week 1** - **change from baseline** | Mean | -1.0 | -0.5 |
| | Std Dev | 1.7 | 1.1 |
| | Minimum | -6 | -3 |
| | Median | -0.9 | -0.3 |
| | Maximum | 2 | 2 |
| **Week 2** | Mean | 3.6 | 4.6 |
| | Std Dev | 2.1 | 1.7 |
| | Minimum | 0 | 2 |
| | Median | 3.5 | 4.4 |
| | Maximum | 10 | 9 |
| **Week 2** - **change from baseline** | Mean | -2.1 | -1.1 |
| | Std Dev | 2.0 | 1.9 |
| | Minimum | -8 | -7 |
| | Median | -1.7 | -0.8 |
| | Maximum | 1 | 2 |
| **Week 3** | Mean | 3.8 | 4.4 |
| | Std Dev | 2.2 | 1.9 |
| | Minimum | 0 | 0 |
| | Median | 3.6 | 4.4 |
| | Maximum | 9 | 8 |
| **Week 3** - **change from baseline** | Mean | -2.0 | -1.4 |
| | Std Dev | 2.0 | 1.8 |
| | Minimum | -7 | -6 |
| | Median | -1.6 | -1.1 |
| | Maximum | 1 | 1 |
| **Week 4** | Mean | 3.5 | 4.5 |
| | Std Dev | 2.2 | 2.1 |
| | Minimum | 0 | 1 |
| | Median | 3.4 | 4.0 |
| | Maximum | 9 | 9 |
| **Week 4** - **change from baseline** | Mean | -2.3 | -1.4 |
| | Std Dev | 2.2 | 2.1 |
| | Minimum | -9 | -6 |
| | Median | -1.9 | -0.9 |
| | Maximum | 2 | 2 |
| **Week 5** | Mean | 3.3 | 4.5 |
| | Std Dev | 2.2 | 2.2 |
| | Minimum | 0 | 0 |
| | Median | 3.0 | 4.3 |
| | Maximum | 8 | 9 |
| **Week 5** - **change from baseline** | Mean | -2.5 | -1.3 |
| | Std Dev | 2.2 | 2.1 |
| | Minimum | -9 | -6 |
| | Median | -2.1 | -1.2 |
| | Maximum | 1 | 2 |
| **Week 6** | Mean | 2.6 | 5.1 |
| | Std Dev | 1.8 | 1.6 |
| | Minimum | 1 | 4 |
| | Median | 2.2 | 4.9 |
| | Maximum | 5 | 7 |
| **Week 6** - **change from baseline** | Mean | -2.2 | -0.2 |
| | Std Dev | 1.1 | 1.7 |
| | Minimum | -3 | -3 |
| | Median | -2.3 | 0.3 |
| | Maximum | -1 | 1 |
| **End Point** | Mean | 3.4 | 4.6 |
| | Std Dev | 2.2 | 2.2 |
| | Minimum | 0 | 1 |
| | Median | 3.5 | 4.0 |
| | Maximum | 8 | 10 |
| **End Point** - **change from baseline** | Mean | -2.3 | -1.1 |
| | Std Dev | 2.2 | 2.0 |
| | Minimum | -9 | -5 |
| | Median | -1.8 | -0.9 |
| | Maximum | 1 | 2 |

Statistical analysis of this data is shown in Table 4.

**Table 4:**

| **THC:CBD (27mg/ml: 25mg/ml)** | | **Placebo** | | | | |
|---|---|---|---|---|---|---|
| LS Mean | s.e. | LS Mean | s.e. | Difference | 95% CI | p-value |
| -2.31 | 0.35 | -1.14 | 0.38 | -1.17 | [-2.00, -0.14] | 0.027 |

The LS Mean figure is the mean change from the baseline adjusted score, a negative difference indicates a benefit.

Tables 3 and 4 demonstrate that the administration of THC:CBD (27mg/ml:25mg/ml) to patients suffering pain in rheumatoid arthritis results in an improved quality of sleep when compared to the placebo.

### Comparison of Disease Activity Score in patients with rheumatoid arthritis when administered a cannabis based medicine extract containing THC at a concentration of 27mg/ml and CBD at a concentration of 25mg/ml

The efficacy of a cannabis based medicine extract was assessed as described above and the Disease Activity Score for each patient was determined at visits 1 and 4. The data was collated and statistical analysis was undertaken. Tables 5 and 6 illustrate the results.

**Table 5:**

| | | **THC:CBD (27mg/ml:25mg/ml) (N=31)** | **Placebo (N=27)** |
|---|---|---|---|
| **Visit 1** | Mean | 5.88 | 6.00 |
| | Std Dev | 0.95 | 1.03 |
| | Minimum | 4.6 | 3.8 |
| | Median | 5.70 | 6.00 |
| | Maximum | 7.8 | 7.8 |
| **Visit 4** | Mean | 5.00 | 5.90 |
| | Std Dev | 1.09 | 1.10 |
| | Minimum | 3.0 | 4.0 |
| | Median | 4 .90 | 5.80 |
| | Maximum | 7.1 | 8.2 |
| **Change from Visit 1** | Mean | -0.85 | -0.16 |
| | Std Dev | 0.81 | 0.98 |
| | Minimum | -2.7 | -3.0 |
| | Median | -0.70 | 0.05 |
| | Maximum | 0.5 | 1.3 |

Statistical analysis of this data is shown in Table 6.

**Table 6:**

| **THC:CBD (27mg/ml: 25mg/ml)** | | **Placebo** | | | | |
|---|---|---|---|---|---|---|
| LS Mean | s.e. | LS Mean | s.e. | Difference | 95% CI | p-value |
| -0.88 | 0.16 | -0.03 | 0.17 | -0.76 | [-1.23, -0.28] | 0.002 |

The LS Mean figure is the mean change from the baseline adjusted score, a negative difference indicates a benefit.
Tables 5 and 6 demonstrate that the administration of THC:CBD (27mg/ml:25mg/ml) to patients suffering pain in rheumatoid arthritis results in an improved Disease Activity Score when compared to the placebo.

The use of a mixture of THC and CBD, where the cannabinoids are in approximately equal quantities when provided to patients with pain associated with rheumatoid arthritis resulted in a decrease in morning pain at rest. The quality of sleep that was experienced by the patients provided with the mixture of equal quantities of THC and CBD was also shown to improve. The patients who were provided with the medication also experienced a decrease in their pain at present as recorded from a questionnaire. Most significantly of all was the effect the medication had on the patients Disease Activity Score.

The Disease Activity Score is a method used to measure the degree of rheumatoid arthritis experienced by a patient. It involves determination of how swollen and tender 28 different joints are. A blood test is also used as part of the Disease Activity Score to measure the erythrocyte sedimentation rate. This rate is a lab method for determining an acute phase response to inflammation. A global disease activity score based on how the patient is feeling also contributes to an overall figure that is calculated. A composite score of greater than 3.7 is considered to be high.

The significance of the findings of the present invention that the use of an approximately 1:1 combination of THC and CBD are able to decrease the Disease Activity Score in patients with rheumatoid arthritis is great.

### References:

Formukong E. A., Evans A. T. and Evans F.J. (1988) Analgesic and Antiinflammatory activity of constituents of Cannabis sativa L. Inflammation 12(4), 361-371
Holdcroft A. et al. (1997a) Pain relief with oral cannabinoids in familial Mediterranean fever. Anaesthesia 52(5), 483-6
Holdcroft A. et al. (1997b) Clinical trial experience with cannabinoids. Pharm. Sci. 3, 546-550
Klein T. W., Newton C. and Friedman H. (1998) Immunol. Today 19, 373-380
Malfait A M et al (2000) The nonpsychoactive cannabis constituent cannabidiol is an oral anti-arthritic therapeutic in murine collagen-induced arthritis. Proc. Nat. Ac. Sci. 97(17), 9561-9566.

## Claims

1. A combination of cannabinoids cannabidiol (CBD) and delta-9-tetrahydrocannabinol (THC) for use as a disease modifying treatment of rheumatoid arthritis wherein the ratio of CBD:THC is substantially 1:1, and wherein the dose taken by a patient is in the range of 5-25mg of each cannabinoid per day sublingually or buccally.

2. A combination of cannabinoids cannabidiol (CBD) and delta-9-tetrahydrocannabinol (THC) for use as claimed in claim 1, which further comprises treating one or more symptoms of rheumatoid arthritis.

3. A combination of cannabinoids cannabidiol (CBD) and delta-9-tetrahydrocannabinol (THC) for use as claimed in claim 2, wherein the one or more symptoms of rheumatoid arthritis to be treated is pain.

4. A combination of cannabinoids cannabidiol (CBD) and delta-9-tetrahydrocannabinol (THC) for use as claimed in claim 2, wherein the one or more symptoms of rheumatoid arthritis to be treated is inflammation.

5. A combination of cannabinoids cannabidiol (CBD) and delta-9-tetrahydrocannabinol (THC) for use as claimed in claim 2, wherein the one or more symptoms of rheumatoid arthritis to be treated is improvement in quality of sleep.

6. A combination of cannabinoids cannabidiol (CBD) and delta-9-tetrahydrocannabinol (THC) for use as claimed in any of the preceding claims, wherein the ratio of CBD:THC is 0.93:1.

7. A combination of cannabinoids cannabidiol (CBD) and delta-9-tetrahydrocannabinol (THC) for use as claimed in any of the preceding claims, wherein the cannabinoids are formulated with one or more carrier solvents.

8. A combination of cannabinoids cannabidiol (CBD) and delta-9-tetrahydrocannabinol (THC) for use as claimed in claim 7, wherein the one or more carrier solvents are ethanol and / or propylene glycol.

9. A combination of cannabinoids cannabidiol (CBD) and delta-9-tetrahydrocannabinol (THC) for use as claimed in claim 8, wherein the ethanol and propylene glycol are in a ratio of between 4:1 and 1:4.

10. A combination of cannabinoids cannabidiol (CBD) and delta-9-tetrahydrocannabinol (THC) for use as claimed in claim9, wherein the ratio of ethanol to propylene glycol is 1:1.

11. A combination of cannabinoids cannabidiol (CBD) and delta-9-tetrahydrocannabinol (THC) for use as claimed in any of the preceding claims, wherein the cannabinoids are present as a cannabis based medicinal extract (CBME).

12. A combination of cannabinoids cannabidiol (CBD) and delta-9-tetrahydrocannabinol (THC) for use as claimed in any of the preceding claims, wherein the cannabinoids are derived from one or more CBME's.

13. A combination of cannabinoids cannabidiol (CBD) and delta-9-tetrahydrocannabinol (THC) for use as claimed in claim 12, wherein the cannabinoids are formulated to comprise a combination of:
a) a cannabis based medicinal extract which comprises THC at more than 90% of the total cannabinoid content of the extract; and
b) a cannabis based medicinal extract which comprises CBD at more than 90% of the total cannabinoid content of the extract.

14. A combination of cannabinoids cannabidiol (CBD) and delta-9-tetrahydrocannabinol (THC) for use as claimed in any claims 12 to 13, wherein the CBME's are produced by extraction with supercritical or subcritical CO₂.

15. A combination of cannabinoids cannabidiol (CBD) and delta-9-tetrahydrocannabinol (THC) for use as claimed in claim 1, wherein the cannabinoids are pure.

16. A combination of cannabinoids cannabidiol (CBD) and delta-9-tetrahydrocannabinol (THC) for use as claimed in claim 1, wherein the cannabinoids are synthetic.

## Patentansprüche

1. Kombination aus den Cannabinoiden Cannabidiol (CBD) und Delta-9-Tetrahydrocannabinol (THC) für die Verwendung als krankheitsmodifizierende Behandlung von rheumatoider Arthritis, wobei das Verhältnis von CBD:THC im Wesentlichen 1:1 ist und wobei die Dosis, die von einem Patienten eingenommen wird, im Bereich von 5-25 mg für jedes Cannabinoid pro Tag sublingual oder bukkal liegt.

2. Kombination aus den Cannabinoiden Cannabidiol (CBD) und Delta-9-Tetrahydrocannabinol (THC) für die Verwendung nach Anspruch 1, die ferner das Behandeln von einem oder mehreren Symptomen rheumatoider Arthritis umfasst.

3. Kombination aus den Cannabinoiden Cannabidiol (CBD) und Delta-9-Tetrahydrocannabinol (THC) für die Verwendung nach Anspruch 2, wobei das eine oder die mehreren Symptome rheumatoider Arthritis, die behandelt werden sollen, Schmerz ist.

4. Kombination aus den Cannabinoiden Cannabidiol (CBD) und Delta-9-Tetrahydrocannabinol (THC) für die Verwendung nach Anspruch 2, wobei das eine oder die mehreren Symptome rheumatoider Arthritis, die behandelt werden sollen, Entzündung ist.

5. Kombination aus den Cannabinoiden Cannabidiol (CBD) und Delta-9-Tetrahydrocannabinol (THC) für die Verwendung nach Anspruch 2, wobei das eine oder die mehreren Symptome rheumatoider Arthritis, die behandelt werden sollen, eine Verbesserung der Qualität des Schlafs ist.

6. Kombination aus den Cannabinoiden Cannabidiol (CBD) und Delta-9-Tetrahydrocannabinol (THC) für die Verwendung nach einem der vorhergehenden Ansprüche, wobei das Verhältnis von CBD:THC 0,93:1 ist.

7. Kombination aus den Cannabinoiden Cannabidiol (CBD) und Delta-9-Tetrahydrocannabinol (THC) für die Verwendung nach einem der vorhergehenden Ansprüche, wobei die Cannabinoide mit einem oder mehreren Trägerlösungsmitteln formuliert sind.

8. Kombination aus den Cannabinoiden Cannabidiol (CBD) und Delta-9-Tetrahydrocannabinol (THC) für die Verwendung nach Anspruch 7, wobei das eine oder die mehreren Trägerlösungsmittel Ethanol und/oder Propylenglykol sind.

9. Kombination aus den Cannabinoiden Cannabidiol (CBD) und Delta-9-Tetrahydrocannabinol (THC) für die Verwendung nach Anspruch 8, wobei das Ethanol und das Propylenglykol in einem Verhältnis von zwischen 4:1 und 1:4 verwendet werden.

10. Kombination aus den Cannabinoiden Cannabidiol (CBD) und Delta-9-Tetrahydrocannabinol (THC) für die Verwendung nach Anspruch 9, wobei das Verhältnis von Ethanol zu Propylenglykol 1:1 ist.

11. Kombination aus den Cannabinoiden Cannabidiol (CBD) und Delta-9-Tetrahydrocannabinol (THC) für die Verwendung nach einem der vorhergehenden Ansprüche, wobei die Cannabinoide als Heilmittelextrakt auf Cannabisbasis (cannabis based medicinal extract - CBME) vorliegen.

12. Kombination aus den Cannabinoiden Cannabidiol (CBD) und Delta-9-Tetrahydrocannabinol (THC) für die Verwendung nach einem der vorhergehenden Ansprüche, wobei die Cannabinoide von einem oder mehreren CBMEs stammen.

13. Kombination aus den Cannabinoiden Cannabidiol (CBD) und Delta-9-Tetrahydrocannabinol (THC) für die Verwendung nach Anspruch 12, wobei die Cannabinoide so formuliert sind, dass sie eine Kombination der Folgenden umfassen:
a) ein Heilmittelextrakt auf Cannabisbasis, das THC umfasst, das mehr als 90 % des Gesamtcannabinoidgehalts des Extrakts ausmacht; und
b) ein Heilmittelextrakt auf Cannabisbasis, das CBD umfasst, das mehr als 90 % des Gesamtcannabinoidgehalts des Extrakts ausmacht.

14. Kombination aus den Cannabinoiden Cannabidiol (CBD) und Delta-9-Tetrahydrocannabinol (THC) für die Verwendung nach einem der Ansprüche 12 bis 13, wobei die CBMEs durch die Extraktion mit überkritischem oder unterkritischem Kohlendioxid erzeugt werden.

15. Kombination aus den Cannabinoiden Cannabidiol (CBD) und Delta-9-Tetrahydrocannabinol (THC) für die Verwendung nach Anspruch 1, wobei die Cannabinoide rein sind.

16. Kombination aus den Cannabinoiden Cannabidiol (CBD) und Delta-9-Tetrahydrocannabinol (THC) für die Verwendung nach Anspruch 1, wobei die Cannabinoide synthetisch sind.

## Revendications

1. Combinaison des cannabinoïdes cannabidiol (CBD) et delta-9-tétrahydrocannabinol (THC) pour utilisation comme traitement de fond de la polyarthrite rhumatoïde dans laquelle le rapport de CBD:THC vaut sensiblement 1:1, et la dose prise par un patient se trouvant dans la plage de 5 à 25 mg de chaque cannabinoïde par jour par voie sublinguale ou buccale.

2. Combinaison des cannabinoïdes cannabidiol (CBD) et delta-9-tétrahydrocannabinol (THC) pour utilisation selon la revendication 1, qui comprend en outre le traitement d'un ou plusieurs symptômes de la polyarthrite rhumatoïde.

3. Combinaison des cannabinoïdes cannabidiol (CBD) et delta-9-tétrahydrocannabinol (THC) pour utilisation selon la revendication 2, lesdits un ou plusieurs symptômes de la polyarthrite rhumatoïde à traiter étant la douleur.

4. Combinaison des cannabinoïdes cannabidiol (CBD) et delta-9-tétrahydrocannabinol (THC) pour utilisation selon la revendication 2, lesdits un ou plusieurs symptômes de la polyarthrite rhumatoïde à traiter étant l'inflammation.

5. Combinaison des cannabinoïdes cannabidiol (CBD) et delta-9-tétrahydrocannabinol (THC) pour utilisation selon la revendication 2, lesdits un ou plusieurs symptômes de la polyarthrite rhumatoïde à traiter étant l'amélioration de la qualité du sommeil.

6. Combinaison des cannabinoïdes cannabidiol (CBD) et delta-9-tétrahydrocannabinol (THC) pour utilisation selon l'une quelconque des revendications précédentes, le rapport de CBD:THC valant 0,93:1.

7. Combinaison des cannabinoïdes cannabidiol (CBD) et delta-9-tétrahydrocannabinol (THC) pour utilisation selon l'une quelconque des revendications précédentes, lesdits cannabinoïdes étant formulés avec un ou plusieurs solvants vecteurs.

8. Combinaison des cannabinoïdes cannabidiol (CBD) et delta-9-tétrahydrocannabinol (THC) pour utilisation selon la revendication 7, lesdits un ou plusieurs solvants vecteurs étant l'éthanol et/ou le propylène glycol.

9. Combinaison des cannabinoïdes cannabidiol (CBD) et delta-9-tétrahydrocannabinol (THC) pour utilisation selon la revendication 8, l'éthanol et le propylène glycol étant dans un rapport compris entre 4:1 et 1:4.

10. Combinaison des cannabinoïdes cannabidiol (CBD) et delta-9-tétrahydrocannabinol (THC) pour utilisation selon la revendication 9, le rapport de l'éthanol sur le propylène glycol valant 1:1.

11. Combinaison des cannabinoïdes cannabidiol (CBD) et delta-9-tétrahydrocannabinol (THC) pour utilisation selon l'une quelconque des revendications précédentes, lesdits cannabinoïdes étant présents sous la forme d'un extrait médicinal à base de cannabis (CBME).

12. Combinaison des cannabinoïdes cannabidiol (CBD) et delta-9-tétrahydrocannabinol (THC) pour utilisation selon l'une quelconque des revendications précédentes, lesdits cannabinoïdes provenant d'un ou plusieurs CBME.

13. Combinaison des cannabinoïdes cannabidiol (CBD) et delta-9-tétrahydrocannabinol (THC) pour utilisation selon la revendication 12, lesdits cannabinoïdes étant formulés pour comprendre une combinaison de :
a) un extrait médicinal à base de cannabis qui comprend du THC à plus de 90 % de la teneur totale en cannabinoïdes de l'extrait ; et
b) un extrait médicinal à base de cannabis qui comprend du CBD à plus de 90 % de la teneur totale en cannabinoïdes de l'extrait.

14. Combinaison des cannabinoïdes cannabidiol (CBD) et delta-9-tétrahydrocannabinol (THC) pour utilisation selon l'une quelconque des revendications 12 à 13, lesdits CBME étant produits par extraction avec du CO₂ supercritique ou sous-critique.

15. Combinaison des cannabinoïdes cannabidiol (CBD) et delta-9-tétrahydrocannabinol (THC) pour utilisation selon la revendication 1, lesdits cannabinoïdes étant purs.

16. Combinaison des cannabinoïdes cannabidiol (CBD) et delta-9-tétrahydrocannabinol (THC) pour utilisation selon la revendication 1, lesdits cannabinoïdes étant synthétiques.
